# EUROPEAN PATENT APPLICATION

(11) **EP 4 260 796 A1**
(43) Date of publication of application: **18.10.2023**
(21) Application number: 22275046.5
(22) Date of filing: 13.04.2022
(51) Int. Cl.: A61B 3/12, A61B 3/10

(54) **SCANNING DISPLAY DEVICE**

(71) Applicant: BAE SYSTEMS plc, London SW1Y 5AD (GB)
(72) Inventor: The designation of the inventor has not yet been filed
(74) Representative: BAE SYSTEMS plc Group IP Department

(57) **Abstract**

A scanning display device configured to scan a retina of an eye of a user, the scanning comprising: a light source for generating a collimated beam; optics for directing the collimated beam towards a reflective combiner configured to reflect the collimated beam to a retina; a scanning element for scanning the collimated beam over at least two axes onto the retina via the reflective combiner located between the scanning element and the retina; wherein the collimated beam has a predetermined diameter based on a size of an eye pupil of the user

## Description

### FIELD

The present invention relates to a scanning display device particularly, but not exclusively for a visor on a helmet or a head mounted system.

### BACKGROUND

A retinal scanning display device allows a light beam to be projected directly onto a retina of a user, and allows the projected light beam to be scanned onto the retina, to thereby display an image onto the retina.

Such retinal scanning display devices are, in general, configured to include a light source emitting a light beam, a scanner scanning the light beam emitted from the light source onto a retina and a light exit at which the light beam scanned by the scanner exits the retinal scanning display device into the eye. In most situations these display devices are head mounted. The retinal scanning display devices can be bulky and may give rise to discomfort if the user is wearing the device for a significant time

Typical retinal scanning display devices can also easily present misinformation to a user: if the eye moves whilst the light beam is being projected the image may not be true and include aberrations or worse. In addition, conditions may prevent a true image being generated. This could potentially lead to accidents, particularly where the user is piloting or driving a vehicle.

In addition, typical devices have a small pupil which means that if the eye moves during projection the image generated on the retina is moving and the eye is not fooled into seeing the image as intended. This has made retinal scanning display devices a non-preferred option in applications where precision is needed for example when piloting a vehicle such as an aircraft.

A need exists to find a simple retinal scanning display device which is lightweight and comfortable to wear. A further need is for a retinal scanning display device which presents a true image to the user and reduces the risks that the image contains misinformation.

### SUMMARY

According to an aspect of the present invention, there is provided a scanning display device configured to scan a retina of an eye of a user, the scanning comprising: a light source for generating a beam; optics for directing the beam towards a reflective combiner configured to reflect the beam to a retina; a scanning element for scanning the beam over at least two axes onto the retina via the reflective combiner located between the scanning element and the retina; wherein the beam has a predetermined diameter based on a size of an eye pupil of the user.

In an aspect the beam is a collimated beam.

In an aspect the predetermined diameter is selected to be bigger than the eye pupil

In an aspect the predetermined diameter is in a range of between about 3mm and about 20mm.

In an aspect the predetermined diameter is about 10mm.

In an aspect the reflective combiner is located between the scanning element and the retina.

In an aspect a percentage of reflectivity of the reflective combiner varies based on the application and is between 0% and 100%.

In an aspect the percentage of reflectivity of the reflective combiner is 50%.

In an aspect the percentage of reflectivity of the reflective combiner if there is no need to see the outside world is 100%.

In an aspect the device configured to present a combination of a real-world view and virtual information via the scanning display device in proportions based on the reflectivity.

In an aspect the scanning display device is configured to present virtual information.

In an aspect the virtual information comprises symbology associates with a real-world scene.

In an aspect the scanning element includes an elevation scanning element and an azimuth scanning element.

In an aspect the elevation scanning element comprises a scanning mirror.

In an aspect the azimuth scanning element further comprises a light pipe.

According to an aspect of the present invention, there is provided a helmet comprising a scanning display device according to another aspect of the invention.

In an aspect the helmet further comprises a visor.

In an aspect the visor is configured to provide the reflective combiner located between the scanning element and the retina.

In an aspect the visor is configured to provide the reflective combiner) located between the scanning element and the retina.

According to an aspect of the present invention, there is provided a method to provide virtual information directly to a retina of an eye of a user the method comprising: modulating a light beam; scanning the modulated beam over at least two directions to produce a modulated beam having a predetermined diameter; directing the modulate beam to the retina via a reflective combiner such that the beam raster scans the retina to project an image directly onto the retina.

In an aspect the modulated beam is a collimated beam.

In an aspect further comprising presenting symbology which can aid a user to drive a vehicle

In an aspect further comprising conditioning the beam.

In an aspect further comprising providing a real world view via the reflective combiner.

In an aspect further comprising varying a percentage of reflectivity of the reflective combiner based on the application and to be between 0% and 100%.

In an aspect further comprising presenting a combination of a real-world view and virtual information via a scanning display device in proportions based on the reflectivity.

In an aspect further comprising providing the reflective combiner as a visor of a helmet incorporating a scanning display device carrying out the method.

### BRIEF DESCRIPTION OF THE FIGURES

Embodiments of the invention will now be described by way of example only with reference to the figures, in which:
Figure 1 shows a schematic view of a helmet according to an aspect of the present invention;
Figure 2 shows a representation of how a retinal scanning display device projects light to an eye;
Figure 3 shows an optical diagram of a retinal scanning display device according to an aspect of the present invention;
Figure 4 is a flow diagram illustrating how symbology is presented to an eye of the user.

### DETAILED DESCRIPTION

The present invention relates to a retinal scanning display device for use in a head mounted device. The retinal scanning display device is capable of scanning a display across the eye at sufficient speed to exploit the persistence of the retina to form an illusion of a constant display. In this way a user is provided with at least one of a real image, a virtual image and an augmented image having real and virtual components. The retinal scanning display device is configured to scan a beam of light across the eye to form the image on the eye's retina. The beam is modulated and/or collimated as an example and as is described below.

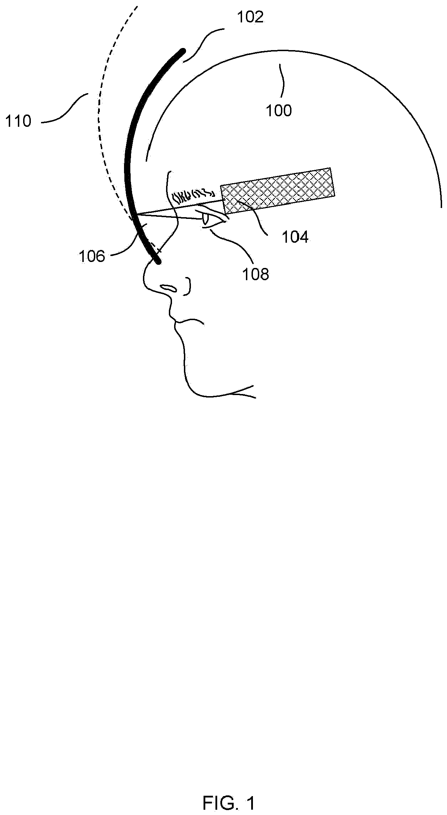

Figure 1 shows a helmet 100 equipped with a visor 102 and including side mounted optics 104 which will be described in greater detail below. The visor forms part of the optics for projecting light 106 from the side mounted optics 104 to an eye 108 of a user. Figure 1 also shows a nominal position of an existing visor position 110 for demonstrating that the present invention enables a more compact overall helmet due to the nature of the optics and that the visor 102 conforms more closely to the standard shape of the helmet 100.

The side mounted optics 104 also provide certain advantages in that the there is less obstruction to the view of the user and the positioning is aligned more closely to the centre of gravity of the helmet 100 giving rise to less physical strain for the user.

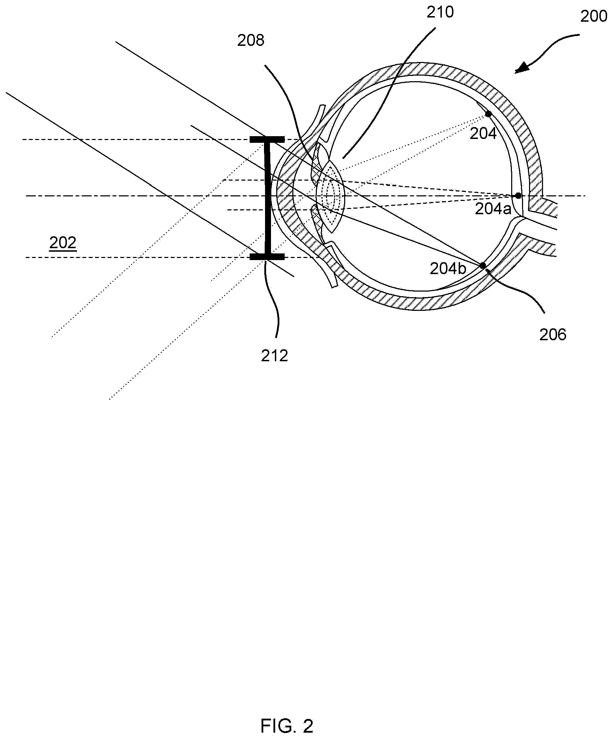

Referring to figure 2, when the eye 200 is illuminated with a beam 202 such as a collimated beam, a spot 204 is formed on the retina 206. The figure shows spots 204, 204a, 204b being formed at different field angles. The collimated beam enters the eye through iris 208 which acts as an aperture and is focused onto the retina by lens 210. The beam is scanned across a range of angles and the brightness is modulated such that the brain will recognize the projected light as a real image. The modulation is an adjustment in instantaneous brightness between 0 (zero) to the full display brightness. This will cause persistence in the retina to generate the illusion of an image once the raster scan is completed. The angle of the scan directly relates to the display FOV. The smallest angle of scan to generate a pseudo image is the smallest angle to which the eye can perceive an image, for practical purposes the would be about 0.05 degrees. The scanning comprises raster scanning the collimated beam over the retina and has an effective pupil 212 into the eye.

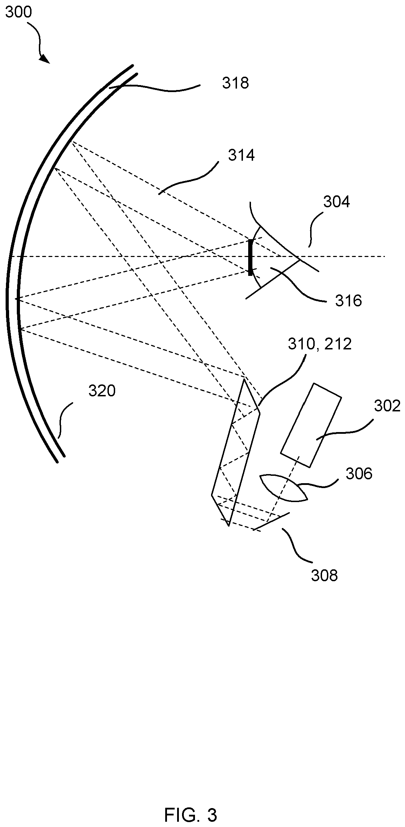

The beams from different field angles comprise colour beams of bright light from Light Emitting Diodes (LEDs), lasers or any other appropriate source. The colours could be all the same or different. The beams are then conditioned through a lens system and directed to the visor via a scanning element to generate the display field angles as illustrated with reference to figure 3.

Figure 3 shows a schematic diagram of a retinal scanning display device 300. The device 300 includes a modulated light source 302 which is projected onto the eye 304 of a user via optics. The optics include correction optics 306 which comprises one or more lenses or any other appropriate optical element or elements. In addition, a scanning optical component 308 comprises an elevation scanning element which comprises a mirror or other suitable scanning display device. A light pipe 310 which also comprises an azimuth scanning element 312. The scanning is over two axes in a raster scanning process and controlled by a controller (not shown). The controller adapts the presentation of the light to the eye to ensure the required information is presented. The controller adapts the presentation of information so that the combination of the real world view and any virtual information are presented at a focal point that avoids unnecessary eye strain. This will generally mean that the real and virtual information appear to come from the same position. The modulated light source 302, the correction optics 306, the scanning optical components 308, 310 and 312 are located in the side mounted optics 104 shown in figure 1 or any other optics module for use with the helmet or head mounted device.

The optics are made from lightweight materials to avoid any strain on the user. The materials include aluminium, plastics, carbon composites and rubbers.

The light from the optics is in the form of collimated light bundles 314 having a diameter d. The diameter d is selected to be of the order of 10 mm which is an approximate size of the pupil 316 of the eye 304. The diameter is variable and can be greater or smaller than 10 mm and be with a range of 3mm to 20mm directed to a visor 318 of the helmet. The diameter "d" at 10mm is selected as it is bigger than the eye pupil which in daytime is about 3mm. This will allow the eye to be able to move in its pupil plane without losing the display source. In practice the eye can move up to 5mm in the pupil plane from the perfect alignment position before the display starts to cut off and eventually disappear when the display pupil is outside of the pupil of the eye. The larger the pupil the larger the challenge to provide an appropriate optical schema.

This pupil size is large compared with previous retinal scanning display devices. The image is focussed on the retina over a number of eye rods. By configuring the beams to achieve this the movement of the eye has less impact as the projected beam illuminates a consistent portion of the retina even if the eye moves.

The visor 318 includes a partially mirrored inner surface 320 which directs the modulated collimated beams 314 towards the pupil 316 and in conjunction with the optics, is configured to scan the beam of modulated collimated light across the eye to form the image on the eye's retina (not shown in figure 3). The partial mirror allows external images to also be directed to the eye of the user. This could be a real environment or scene viewable by the user based on location and position. The percentage of reflectivity of surface 320 will depend on the application. The lower the percentage the dimmer any the virtual information will be, and the outside world will appear brighter A typical reflectivity would be about 50% where equal weight is given to the real and virtual information or images. If there is no need to see the outside world then 100% reflection could be used.

The visor could be replaced with another reflective combiner other than the visor in applications where there is no visor. An example may be goggles having at least a portion of the internal surface including a partial mirrored surface for directing the collimated beam to the retina. The partially mirrored inner surface is configured to reflect a percentage for the incident collimated beam based on the application.

External light from a scene or a cockpit can also enter the eye through the visor or reflective combiner so the user can view a real image and the collimated scanned modulated beams.

The real image shows a real world representation of the scene or environment in which the user is located. In the case of a pilot in a vehicle this includes the view from outside the vehicle and any controls within the cockpit or driving position of the user.

The collimated scanned modulated beams display a virtual image of information that is germane to the activity of the user. The virtual information comprises symbology, vehicular and scene information. The vehicular and scene information or data includes position, location, speed, temperature, radar or other sensor data which is from the scene or relating to the vehicle. It will be appreciated that the above information or data can be of any type coming from any appropriate sensor.

The retinal scanning display device could be used in different environments and is not limited for use on a helmet or head worn device. In the situation of a head worn device this could be a helmet and visor as shown in figure 1, or could be any other type of head worn device. The reflective combiner can form part of the retinal scanning display device or can be part of a helmet or goggles that has been configured to act as the reflective combiner for the retinal scanning display device.

In the case of head worn devices the side mounted optics could be located in different locations on the helmet although the optimal location is side mounted as shown, to reduce possible strain to the user.

The materials are just examples and other appropriate materials could be used.

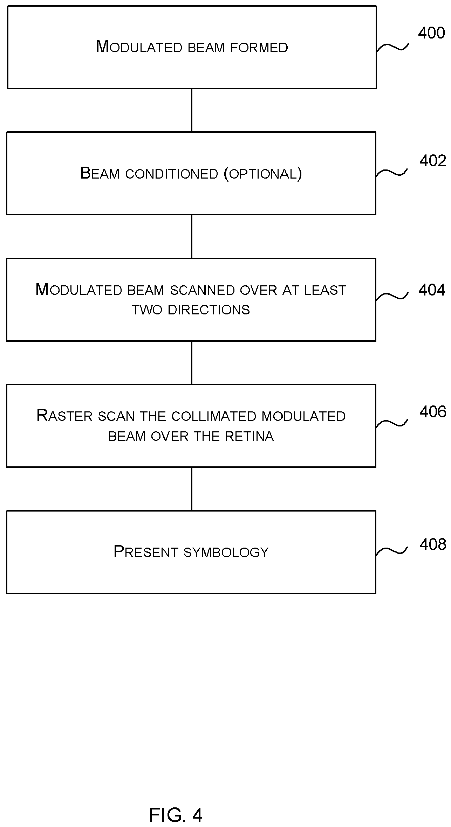

Figure 4 shows a flow diagram illustrating how a retina scanning display device operates. A beam such as a modulated beam as an example, is formed 400. Optionally the beam is conditioned 402. The beam is scanned 404 over at least two directions to produce a collimated modulated beam having a predetermined diameter. Direct 406 the collimated modulate beam to the retina via a reflective combiner such that the beam raster scans the retina to project an image directly onto the retina. The raster scan presents symbology which can contain aid to help the user to drive the vehicle and/or sensor data 408 relating to the activities of a user or relating to the user's circumstances.

Symbology can include at least one or more of the following:
- Signs and symbols
- Data from sensors
- Processed data from sensors
- Combinations of sensor data
- Military symbology
- Vehicle related symbology
- Scene related symbology
- Location and positioning symbology
- Map symbology
- Speed and velocity symbology

It should be noted that the light imaged in the present invention in the real or virtual information can be outside optical frequencies and include infrared, ultra-violet and any other appropriate frequencies.

It will be appreciated the invention is described in as described above, but many variations and alternatives will be evident to the skilled person.

## Claims

1. A scanning display device (300) configured to scan a retina of an eye (304) of a user, the scanning comprising:
a light source (302) for generating a beam (314);
optics (306) for directing the beam towards a reflective combiner (320) configured to reflect the beam to a retina;
a scanning element (308, 310, 312) for scanning the beam over at least two axes onto the retina via the reflective combiner (320) located between the scanning element and the retina;
wherein the beam has a predetermined diameter (d) based on a size of an eye pupil of the user.

2. The scanning display device according to claim 1, wherein the beam is a collimated beam.

3. The scanning display device according to claim 1 or claim 2, wherein the predetermined diameter (d) is selected to be bigger than the eye pupil

4. The scanning display device according to any one of the preceding claims, wherein the predetermined diameter (d) is in a range of between about 3mm and about 20mm.

5. The scanning display device according to any one of the preceding claims, wherein the predetermined diameter (d) is about 10mm.

6. The scanning display device according to any one of the preceding claims, further comprising the reflective combiner (320) located between the scanning element and the retina.

7. The scanning display device according to any one of the preceding claims, wherein a percentage of reflectivity of the reflective combiner (320) varies based on the application and is between 0% and 100%.

8. The scanning display device according to claim 7 further configured to present a combination of a real-world view and virtual information via the scanning display device in proportions based on the reflectivity.

9. The scanning display device according to any preceding claim, wherein the scanning display device is configured to present virtual information.

10. The scanning display device according to claim wherein the virtual information comprises symbology associated with a real-world scene.

11. A helmet (100) comprising a scanning display device (300) according to any one of the preceding claims.

12. The helmet of claim 11, wherein the helmet further comprises a visor (102).

13. A visor for use with the scanning display device according to any one of claims 1 to 10, wherein the visor is configured to provide the reflective combiner (320) located between the scanning element and the retina.

14. A method to provide virtual information directly to a retina of an eye of a user the method comprising:
modulating a light beam (400);
scanning (404) the modulated beam over at least two directions to produce a modulated beam having a predetermined diameter (d);
directing (406) the modulate beam to the retina via a reflective combiner such that the beam raster scans the retina to project an image directly onto the retina.

15. The method according to claim 14, further comprising producing the modulated beam as a collimated modulated beam.
